# EUROPEAN PATENT APPLICATION

(11) **EP 2 090 285 A1**
(43) Date of publication of application: **19.08.2009**
(21) Application number: 08002963.0
(22) Date of filing: 18.02.2008
(51) Int. Cl.: A61K 8/35, A61Q 19/10, A61P 17/10, A61Q 19/00

(54) **Means and methods for controlling commensales**

(71) Applicant: B.R.A.I.N. Biotechnology Research and Information Network AG, 64673 Zwingenberg (DE)
(72) Inventor: The designation of the inventor has not yet been filed
(74) Representative: Vossius & Partner

(57) **Abstract**

The present invention relates to a cosmetic composition for the cleaning of the skin, said composition comprising or consisting of one or more compounds of formula (I) 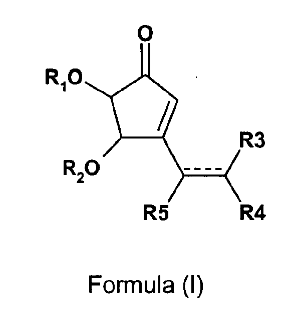 wherein - - - - represents a single bond or double bond; R₁ and R₂ are independently selected from H, linear or branched C₁ to C₄ alkyl, linear or branched C₁ to C₄ alkenyl, linear or branched C₁ to C₄ alkinyl, and linear or branched C₁ to C₄ alkanoyl; R₄ is selected from H, linear or branched C₁ to C₄ alkyl, linear or branched C₁ to C₄ alkenyl, and linear or branched C₁ to C₄ alkinyl; and R₃ and R₅ are independently selected from H, OH and halogen, or R₃ and R₅ together are O to form an epoxid, wherein - - - - represents a single bond in case R₃ and R₅ together are O.

## Description

This invention relates to a cosmetic composition for the cleaning of the skin, said composition comprising or consisting of one or more compounds of formula (I) 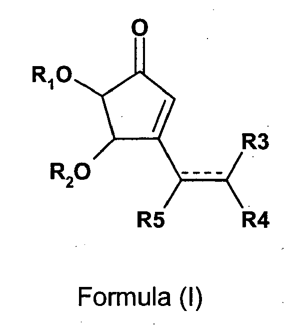 wherein ---- represents a single bond or double bond; R₁ and R₂ are independently selected from H, linear or branched C₁ to C₄ alkyl, linear or branched C₁ to C₄ alkenyl, linear or branched C₁ to C₄ alkinyl, and linear or branched C₁ to C₄ alkanoyl; R₄ is selected from H, linear or branched C₁ to C₄ alkyl, linear or branched C₁ to C₄ alkenyl, and linear or branched C₁ to C₄ alkinyl; and R₃ and R₅ are independently selected from H, OH and halogen, or R₃ and R₅ together are O to form an epoxid, wherein ---- represents a single bond in case R₃ and R₅ together are O.

In this specification, a number of documents including patent applications and manufacturer's manuals is cited. The disclosure of these documents, while not considered relevant for the patentability of this invention, is herewith incorporated by reference in its entirety.

Commensalism is a term employed in ecology and describes a relationship between two living organisms where one benefits and the other is not significantly harmed or helped. The human skin is populated by various commensales including bacteria which in general are not harmful. Nevertheless, it has been considered and is being considered desirable to control the presence and/or the proliferation of commensales of the human or animal skin. It has been observed that in the absence of such controlling under certain conditions commensales may turn into causative agents of undesirable conditions including inflammatory conditions. If not attended to, such conditions may further aggravate and turn into diseases requiring medical treatment. By controlling commensales of the human or animal skin by cosmetic treatment including cleaning and/or disinfection, the likelihood of the development of undesirable skin conditions and skin diseases can be significantly reduced. The choice of the cosmetically active constituents of the cosmetic product depends on the type of commensales which are to be controlled.

In the following, the pertinent prior art is briefly reviewed. This includes a discussion of the undesirable conditions and diseases which may arise in the absence of controlling commensales or in case of insufficient controlling.

Acne is an undesirable condition of the skin, caused by changes in skin structures, i.e. hair follicles and its associated sebaceous gland. Sebaceous glands secrete an oily substance called sebum made of lipids and debris of dead fat producing cells into the upper part of the follicles. In each gland, sebaceous cells move toward the centre of the gland, lipid synthesis within the cells continues until they rupture and lipid is expelled into the excretory stream of the gland. Acne develops as a result of blockages in follicles. Formation of a plug of keratin and sebum is the earliest change. With the onset of adrenarche and an increased androgen production the enlargement of sebaceous glands and an increase in sebum production occurs. When the follicular canal becomes blocked, a microcomedone is formed. The primary manifestation of acne is the closed comedone, which are small lesions of the follicle that are often without a visible central plug. Closed comedones (whiteheads) are non-inflammatory acne lesions. Open comedones (blackheads) consist of small follicular lesions having a central black keratin plug as a result of oxidation of melanin pigment. Open comedones develop from closed comedones as the orifice dilates. The open comedone is not an inflammatory lesion unless traumatized, i.e. picked at, by the affected person. Comedones, either open or closed, are non-inflammatory.

In these conditions the naturally occurring largely commensally bacteria *Propionibacterium acnes* can cause inflammation, leading to inflammatory lesions (papules, infected pustules, or nodules) in the dermis around the microcomedone or comedones. Papules appear when lipases from *P. acnes* metabolize triglycerides into free fatty acids, which irritate the follicular wall. Pustules occur when active *P*. *acnes* infections cause inflammation within the follicle. Nodules and cysts occur when rupture of follicles due to inflammation, physical manipulation or harsh rubbing releases free fatty acids, bacteria, and keratin into tissue, triggering soft-tissue inflammation.

Acne vulgaris is the most common dermatological disorder with 45 million people having this disorder solely in the US, resulting in 5 - 6 million physician visits annually. In the US alone, the estimated annual costs of acne-related healthcare range from 1 billion to $2.2 billion, with approximately $100million spent on over-the-counter products (Del Rosso, 2006). Although acne is not a life-threatening disorder of the skin, it can have serious physiological and socioeconomic consequences that affect a patient's quality of life.

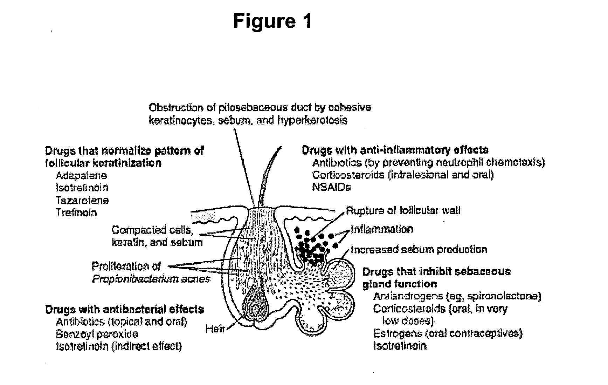

Known treatments are directed at (1) reducing sebaceous gland function (2) normalizing the pattern of follicular keratinisation (3) decreasing the *P.acnes* population and/or decreasing the generation of inflammatory substances by the bacterial population and (4) producing non-inflammatory effects; see Figure 1.

Topical retinoids such as tretoin primarily function by correcting altered patterns of keratinisation. Oral isotretinoin primarily functions by decreasing sebaceous gland activity. Antibiotic therapies such as oral minocycline or topical clindamycine primarily function by reducing the numbers or activity of *P. acnes.* Furthermore, steroids can be injected into acne lesions to produce an anti-inflammatory effect. Acne usually is treated with combination therapy to address its multifactorial pathophysiology (Del Rosso, 2006).

Mild inflammatory acne is treated with topical benzoyl peroxide, topical antibiotics (e.g. erythromycin, clindamycin) and/or glycolic acid. Combinations of these agents may help limit development of resistance. None have significant adverse effects other than drying and irritation and rare allergic reactions to benzoyl peroxide. For a dermal delivery and to localize compounds within the skin to enhance the local effect vehicles such as adhesive hydrogel patches containing triclosan are discussed as anti-acne dosage forms (Lee et al, 2003). Triclosan, (2,4,4'-Trichloro-2'-hydroxy diphenyl ether, Ciba Specialty Chemicals) is a broad spectrum antibacterial/antimicrobial agent. As a result of its bacteriostatic activity against a wide range of both Gram-negative and Gram-positive bacteria it has found increasing and recent popular use in personal care products, i.e. - toothpaste, deodorant soaps, cosmetics and anti-microbial creams etc.

Shortly after introducing topical formulations of antibiotics in the early 1980s, less-sensitive strains of *P. acnes* were reported in the US. Meanwhile a steady increase in clinically significant antibiotic resistance can be observed and *P. acnes* strains with multiple drug resistance were identified (Eady et al, 2003; Ross et al, 2001).

The emergence and spread of erythromycin, clindamycin and/or tetracycline resistant propionibacterial populations threatened their long term viability as anti-acne therapies. A worldwide survey revealed that about 50% of clinical *P. acnes* isolates were resistant to erythromycin and about 20% resistant to tetracycline (Eady et al., 2003). This development clearly underlines that there is an urgent need for novel treatment options.

In the course of the development of new antimicrobial agents, often clinical isolates of sensitive and resistant *P. acnes* strains are tested as well as other commensally Gram-positive bacteria (e.g. *Staphylococcus aureus*). Besides *P. acnes,* other skin bacteria such as *S*. *aureus* or *Streptococcus* spp. demonstrate as well a good susceptibility against new e.g. quinolone antibiotics, thus the observed anti-microbial activities are not acne specific (Nenoff et al., 2004).

In addition to the multiple drug resistance phenomenons, antibiotic-sensitive *P.acnes* strains reveal great tolerance to even high concentrations of antibiotics as a result of its existence in a biofilm matrix. Resistance within the biofilm is related to delayed penetration of antimicrobial agents into the biofilm polysaccharide matrix, the slow growth rate of organisms within the biofilm and the phenotypes of bacteria expressed within the biofilm that are distinct from planktonic (free swimming) cells. In the case of *P.acnes,* an array of extracellular products, including hyaluronidase, proteases, lipases and chemotactic factors for neutrophils, lymphocytes and macrophages are secreted (Burkhart & Burkhart, 2003).

Treatments that target specific components of the biofilm, i.e. to alter the ability of *P*. *acnes* to synthesize the extracellular matrix, or e.g. to reduce the attachment of *P*. *acnes* to the follicular lining are up to now lacking.

This development of (a) multi-resistant *P. acnes* strains and (b) the application of non-acne specific multi component antibiotic treatments clearly underlines that there is an urgent need for novel treatment options.

WO2005/055995 describes terrein as a skin whitening agent or browning inhibitor. W02005/055995 fails to suggest the cosmetic application for cleaning of the skin according to the present invention.

Malmstrøm et al., 2002 describe bioactive metabolites from a marine-derived strain of the fungus *Emericella variecolor.* Terrein is described to exhibit weak antibacterial activity. Activity against a bacterial biofilm is not assayed in Malmstrøm et al., 2002.

The technical problem underlying the present invention was to provide improved or alternative means and methods for controlling commensales.

Accordingly, the present invention relates to a cosmetic composition for the cleaning of the skin, said composition comprising or consisting of one or more compounds of formula (I) 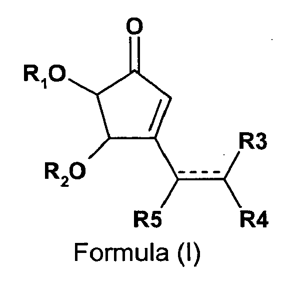 wherein ---- represents a single bond or double bond; R₁ and R₂ are independently selected from H, linear or branched C₁ to C₄ alkyl, linear or branched C₁ to C₄ alkenyl, linear or branched C₁ to C₄ alkinyl, and linear or branched C₁ to C₄ alkanoyl; R₄ is selected from H, linear or branched C₁ to C₄ alkyl, linear or branched C₁ to C₄ alkenyl, and linear or branched C₁ to C₄ alkinyl; and R₃ and R₅ are independently selected from H, OH and halogen, or R₃ and R₅ together are O to form an epoxid, wherein ---- represents a single bond in case R₃ and R₅ together are O.

"Cleaning of the skin" according to the main embodiment is envisaged as a means of controlling impurities of the skin. Accordingly, by applying the cosmetic composition according to the invention, the appearance of the skin is improved.

Cleaning involves bringing the skin to be cleaned into contact with the cosmetic composition according to the invention. Cleaning may further involve mechanic action such as scrubbing, for example, with a pad.

The particular formulation of the cosmetic composition according to the invention is not limited. Envisaged formulations include emulsions, creams, milks, gels such as hydrogels, ointments, suspensions, dispersions, powders, solid sticks, foams, sprays and shampoos. For this purpose, the cosmetic composition according to the invention may further comprise cosmetically acceptable diluents and/or carriers. Choosing appropriate carriers and diluents in dependency of the desired formulation is within the skills of the skilled person. Suitable cosmetically acceptable diluents and carriers are well known in the art and include agents referred to in Bushell et al. (WO 2006/053613). As an example, hydrogel patches may be prepared as described in Lee et al. (2003).

The amount of compounds according to the invention comprised in the cosmetic composition is such that by applying usual amounts of the composition, controlling of commensales of the genus *Propionibacterium* is achieved. Suitable amounts can be determined without further ado by comparing the amounts of commensales prior and after application of the cosmetic composition in dependency of the amount applied. Preferably, such testing starts with lower amounts. Preferably, during a single application, amounts of compounds of the invention between 5µg and 50mg, more preferred between 5µg and 10mg and yet more preferred between 5µg and 5mg, are applied. Preferably, the cosmetic composition of the invention is applied once, twice or three times a day. Preferred concentrations of the compounds according to the invention in the cosmetic composition are between 0,001 weight-% and 10 weight-%, more preferred between 0,001 weight-% and 2 weight-%, and yet more preferred between 0,001 weight-% and 1 weight-%.

Preferred amounts of the cosmetic compositions according to the invention to be applied in a single application are between 0,1 and 10g, more preferred between 0,1 and 1g, most preferred 0,5g.

Preferably, the cosmetic composition according to the invention is left on for a period of time between 1 min and 24 hours, more preferred between 1 hour and 12 hours.

Preferred regions of the skin include the facial skin, the skin on the shoulders, and the skin on the back.

Controlling commensales of the genus *Propionibacterium* by applying the cosmetic composition according to the invention is a cosmetic and in accordance with this embodiment of the invention non-therapeutic treatment.

Preferred substituents R₁ and R₂ are independently selected from methyl, acetyl and hydrogen. In preferred embodiments, both R₁ and R₂ are either methyl, acetyl or hydrogen. Particularly preferred is hydrogen.

Preferably, the substituent R₄ is methyl.

In case a double bond is present, the trans configuration is preferred. In case a double bond is present, it is preferred that both R₃ and R₅ are hydrogen.

In case neither a double bond nor an epoxid is present, it is preferred that one of R₃ and R₅ is OH and the other is selected from halogen and hydrogen.

In a preferred embodiment, the compounds of formula (I) have the following structure:

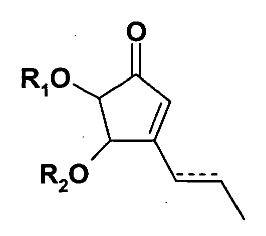

In a more preferred embodiment, the propyl/propenyl side chain is trans-propenyl.

In a yet more preferred embodiment, R1 and R2 are both hydrogen: 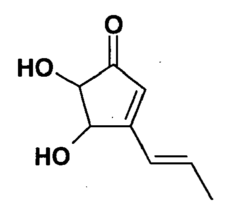 4,5-Dihydroxy-3-(1-propenyl)-2-cyclopenten-1-one. 4,5-Dihydroxy-3-(1-propenyl)-2-cyclopenten-1-one is also known as terrein.

A preferred stereochemistry of the compounds of formula (I) is shown below:

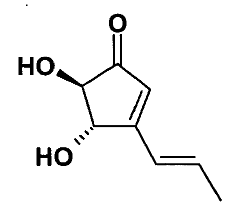

The stereochemistry displayed above is not only preferred for 4,5-Dihydroxy-3-(1-propenyl)-2-cyclopenten-1-one, but also for the compounds of formula (I) in general. Any of the other stereoisomers is also envisaged.

The synthesis of terrein is described, for example, in Lee et al., 2005. Further information on available synthesis routes for terrein and its derivatives as disclosed herein can be found in the Beilstein database as available from Elsevier MDL. To the extent synthetic routes described in the Beilstein database do not explicitly refer to terrein, the skilled person can apply these synthetic routes to terrein and its derivatives by analogy without further ado.

Alternatively, terrein is obtainable by culturing fungi of the genus *Aspergillus* such as *Aspergillus terreus var. terreus* (CBS deposit number CBS 134.60) (Raistrick & Smith, 1935, Clutterbuck et al, 1937), separating the media containing the compounds from the microbial cells and performing purification starting with HPLC-based solid phase extraction, for example on Amberchrom 161c. A H2O/MeOH gradient is then used for elution. The fractions eluting with 80-100% methanol are combined and concentrated to an aqueous residue and lyophilized. The crude material (5.8 g) is subjected to column chromatography on silica gel and eluted with a gradient starting with petrol ether/ethylacetate mixtures in the range 20:1 ∼ 3:1 and finally using chlorofom/methanol mixtures in the range 5:1 ∼ 0:1. The fractions containing the activity against *Propionibacterium* (for assays see the enclosed Examples) are collected. In a specific case, while collecting 320 fractions in total, each fraction having a volume of 5 mL each, terrein was obtained in fractions 71-78. Subsequently, final purification by gel filtration chromatography, for example on Sephadex LH20, may be perfomred. Purification may be monitored by analytical HPLC/MS chromatography. A general strategy for obtaining microbial metabolites such as terrein from fungal sources is also described in He et al, 2005. Furthermore, terrein may also be obtained from *Penicillium*; see Park et al. (2004).

Preferred degrees of purity of the compounds of the invention are at least 90% by weight, more preferred 95%, 96%, 97%, 98%, 99% by weight or above.

The present invention furthermore provides a method of cosmetic treatment comprising the administration of one or more compounds as defined in the main embodiment, wherein said cosmetic treatment is or comprises cleaning of the skin.

In a preferred embodiment, said administration is topical administration. Preferably, said administration is administration to the skin.

The present invention furthermore provides the use of one or more compounds as defined in the main embodiment for cosmetic treatment, wherein said cosmetic treatment is or comprises cleaning of the skin.

In preferred embodiments of the composition, the method and the use according to the invention, said cleaning of the skin comprises removing and/or reducing a biofilm and/or preventing the formation of said biofilm, wherein said biofilm comprises or consists of bacteria of the genus *Propionibacterium.*

The term "biofilm" is known in the art and refers to a mucus layer with microorganisms embedded therein (Nadell et al., 2008; Donlan, 2001). Biofilms are formed by microorganisms at surfaces and adhere to this surface. Accordingly, a biofilm according to the invention may also be referred to as a multicellular surface-bound aggregate. The microorganisms present in biofilms according to the invention either comprise or exclusively consist of bacteria of the genus *Propionibacterium.* In addition to microorganisms, biofilms may comprise water and/or biopolymers secreted by the microorganisms. These polymers are also referred to as "extracellular polymeric substances" (EPS). Any kind of biopolymers may be comprised in the biofilms, including polysaccharides, proteins, lipids and nucleic acids. In particular the presence of polysaccharides gives rise to mucous nature of the biofilm. In the case of *Propionibacterium,* an array of extracellular products, including hyaluronidase, proteases, lipases and chemotactic factors for neutrophils, lymphocytes and macrophages are secreted.

The formation of biofilms renders the control of commensales particularly difficult. For example, and as stated in the introductory part of this specification, the formation of a biofilm often gives rise to resistance to antibiotic agents as a consequence of the delayed penetration of antimicrobial agents into the biofilm polysaccharide matrix, the slow growth rate of organisms within the biofilm and the specific phenotype of the bacteria which develops within the biofilm and is distinct from the phenotype of planktonic cells. As documented in the Examples enclosed herewith, the compounds according to the invention are capable of inhibiting not only planktonic growth of *Propionibacterium,* but also the growth of *Propionibacterium* in biofilms. This capability of the compounds according to the invention is unexpected and very advantageous. As a consequence, the compounds according to the invention solve the technical problem of resistance to antibiotic agents of biofilms comprising *Propionibacterium.*

The prior art reviewed herein above (Malmstrøm et al., loc. cit.) only reported weak to negligible activity of terrein against certain Gram-positive and Gram-negative bacteria including commensales of the skin. Surprisingly it was found that compounds according to the invention exhibit marked activity against bacteria of the genus *Propionibacterium.*

In a further preferred embodiment of the composition, method or use according to the invention, said bacteria of the genus *Propionibacterium* are bacteria of the species *Propionibacterium acnes.*

The present invention furthermore provides a compound or a plurality of compounds, said compound(s) being selected from the compounds as defined in the main embodiment, for the prevention or reduction of biofilm formation on an intracorporeal device and/or for the removal of biofilm from an intracorporeal device, wherein said biofilm comprises or consists of bacteria of the genus *Propionibacterium.*

Prevention or reduction of biofilm formation on an intracorporeal device and removal of biofilm from an intracorporeal device may, depending on the case, be a cosmetic or a medical application.

In a preferred embodiment said intracorporeal device is selected from catheters, implants, endoscopes, drainages, contact lenses and hearing aids.

Endoscopes are diagnostic instruments known in the art and are available for inspection of, for example stomach and intestine. It is known that disinfection of endoscopes is not straightforward, in particular they are not amenable to autoclaving. A drainage is a device which permits to drain secretion from wounds after surgical interventions. Formation of biofilms on drainages may facilitate entry of bacteria into the body, leading to unwanted spread of the bacteria within the body. Biofilms on contact lenses may lead to irritations of the eye and/or impaired vision.

The development or presence of biofilms on intracorporeal devices is a frequently encountered problem which up to date cannot be satisfactorily controlled. Common measures to control biofilms are simple mechanic removal of the biofilm which has to be performed in regular intervals.

In a preferred embodiment, said bacteria of the genus *Propionibacterium* are bacteria of the species *Propionibacterium acnes.*

The present invention furthermore relates to a method of preventing or reducing formation of a biofilm on a device for intracorporeal use and/or of removing of a biofilm from a device for intracorporeal use, wherein said device is not present in a human or animal body, and wherein said biofilm comprises or consists of bacteria of the genus *Propionibacterium,* said method comprising bringing said device into contact with a compound or a plurality of compounds, said compound(s) being as defined in the main embodiment.

The recited "bringing into contact" may be effected such that the compound or the compounds as defined in the main embodiment or a composition comprising the compound(s) is adsorbed or absorbed by the device for intracorporeal use. This may be achieved by using a suitably prepared intracorporeal device. For example, the extracorporeal device may have a roughened surface. The roughened surface may be achieved by using various procedures as known in the art including etching or abrading. The roughened surface permits adsorption or absorption of sufficient amounts of said compounds.

Alternatively or in addition, a composition comprising one or more compounds as defined in the main embodiment may be prepared and used in the method according to the invention, wherein said composition adheres to the surface of any intracorporeal device, for example, as a consequence of surface tension and/or stickiness.

To the extent the above method according to the invention relates to removing of a biofilm, it is envisaged that, in addition to bringing the device into contact with one or more compounds as defined in the main embodiment, mechanic action may be applied, such as scrubbing.

In relation thereto, the present invention also provides the use of a compound or a plurality of compounds, said compound(s) being as defined in the main embodiment for preventing or reducing formation of a biofilm on a device for intracorporeal use and/or for removing of a biofilm from a device for intracorporeal use, wherein optionally said device is not present in a human or animal body, and wherein said biofilm comprises or consists of bacteria of the genus *Propionibacterium.*

In a preferred embodiment, said device for intracorporeal use is selected from catheters, implants, endoscopes, drainages, contact lenses and hearing aids.

In a further preferred embodiment, said bacteria of the genus *Propionibacterium* are bacteria of the species *Propionibacterium acnes.*

The present invention furthermore relates to an intracorporeal device which is coated and/or loaded with one or more compounds, said compound(s) being as defined in the main embodiment.

Preferred intracorporeal devices are listed herein above.

The term "coated" refers to a layer on the surface of the intracorporeal device which comprises or consists of one or more compounds according to the invention. Said layer may cover all or parts of the intracorporeal device.

The term "loaded" refers to an intracorporeal device, wherein said intracorporeal device in its entirety or parts thereof are made of a material, herein also referred to as matrix, which comprises one or more compounds as defined in the main embodiment.

Coating of an intracorporeal device with one or more compound(s) according to the invention may be achieved, for example, by the means described in conjunction with the method of preventing or reducing formation of a biofilm according to the invention further above. Thereby, adsorbing or absorbing of said compound(s) may be achieved.

An intracorporeal device loaded with one or more compounds as defined in the main embodiment may be obtained by producing the intracorporeal device or parts thereof from a material or matrix which permits embedding one or more compounds as defined in the main embodiment therein. This embedding may be such that no or no significant release of the compounds according to the invention occurs. Alternatively or in addition, the matrix may be designed such that a sustained release of one or more compounds as defined in the main embodiment occurs from the matrix. In either case, presence and/or release of one or more compounds as defined above permits the control of biofilm formation.

Suitable matrix materials are well known in the art and include biodegradable as well as non-biodegradable materials. If a biodegradable matrix material is to be used, it will generally be preferred that only parts of the intracorporeal device are made thereof. Polyglycolide, polylactic acid, poly(lactic-co-glycolic acid), polycaprolactone, poly-3-hydroxybutyrate and polydioxanone are envisaged biodegradable matrix materials.

It is also envisaged to cover an intracorporeal device with a layer of biodegradable matrix, wherein said biodegradable matrix comprises one or more compounds as defined in the main embodiment.

The present invention furthermore provides a compound or a plurality of compounds, said compound(s) being selected from the compounds as defined in the main embodiment, for the prevention or treatment of acne, wherein said acne is or involves an infection with bacteria of the genus *Propionibacterium.* In a preferred embodiment, said bacteria of the genus *Propionibacterium* are bacteria of the species *Propionibacterium acnes.*

The latter embodiments refer to medical uses of the compounds as defined in the main embodiment. Acne may manifest itself in forms which are generally considered a disease which in turn requires medical treatment. For the purpose of medical treatment, the compounds as defined in the main embodiment may be formulated as pharmaceutical compositions comprising, in addition to one or more compounds as defined in the main embodiment, pharmaceutically acceptable carriers, diluents and/or excipients. Suitable carriers, diluents and excipients are well known to the person skilled in the art. In a preferred embodiment, the compounds according to the invention, when used in a treatment of acne, are applied topically to affected parts of the skin. Accordingly, it is preferred to use pharmaceutically acceptable diluents, carriers and/or excipients which are commonly used in the preparation of pharmaceutical formulations designed for topical administration to the skin. Suitable pharmaceutically acceptable diluents and carriers for topical administration to the skin are well known in the art and include agents referred to in Bushell et al (WO 2006/053613). Preferred embodiments of the compounds of the invention as disclosed herein above are also preferred for the medical uses according to the invention.

The figures show:
**Figure 1****:** Mode of action of drugs for treating acne. Figure 1 is taken from http://www.merck.com/mmpe/sec10/ch111/ch111b.html.
**Figure 2****:** Inhibition of planktonic growth
**Figure 3****:** Inhibition of biofilm formation
**Figure 4****:** Planktonic growth of *P. acnes*
**Figure 5****:** Biofilm formation of *P. acnes*

The following examples illustrate the invention.

### Example 1: Susceptibility testing of planktonic growing and biofilm cells of P. acnes.

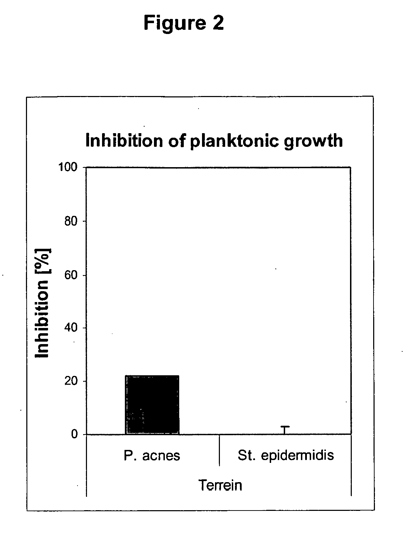

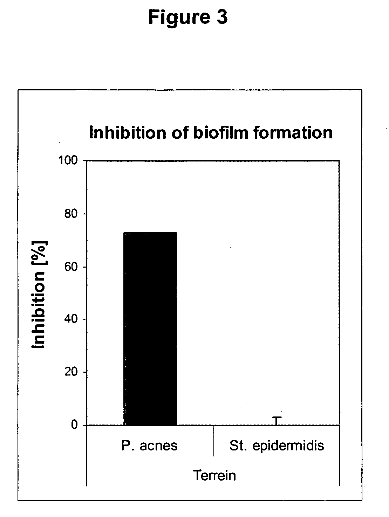

Routine susceptibility testing of *P. acnes* (DSMZ 1897^{T}) is performed in high throughput 96-well microtiter plate format using Wilkins-Chalgren anaerobe broth (CM 0643, Oxoid GmbH, Wesel, Germany) in an anaerobic atmosphere produced using the AnaeroGen^{™} system (10% CO₂ and <1% O₂, Oxoid GmbH, Wesel, Germany) at 37°C. Planktonic growth of cells is determined by measuring turbidity at 580nm in a microtiter plate reader (SpectraMAX, Molecular Devices GmbH, München, Germany). Compounds dissolved in 100% DMSO are added simultaneously to an inoculum of 0.1 OD580 actively growing *P. acnes* cells to give a final concentration of 12.5µg/mL. Growth of untreated planktonic cells started to cease approximately 24 hours after inoculation resulting maximum OD580 values of approximately 0.4. Subsequently, planktonic cells are removed and the biofilm adhering at the bottom of the microtiter plate is been fixed by heat treatment (10min 80°C) and washed before staining with 0.1% crystal violet and quantified at 580nm (CV580) as described in O'Toole, G. A. and Kolter, R. (1998) in a microtiter plate reader (SpectraMAX, Molecular Devices GmbH, München, Germany). Biofilm development reached maximum approximately 24 hours upon inoculation. Data for planktonic proliferation and biofilm formation of *P. acnes* was collected from identical wells of a microtiter plate. Assays were done as quadruples. The effects of test compounds on growth of planktonic cells and formation of biofilm was determined 26-28 hours after inoculation and evaluated by comparison of turbidity (optical density, OD580) and crystal violet staining (CV580) of treated and untreated samples (control) and given as % inhibition (Figures 2 and 3).

### Example 2: Susceptibility testing of planktonic growing and biofilm cells of Staphylococcus epidermidis.

*Staphylococcus epidermidis* occurs frequently on the skin of humans and animals and in mucous membranes. Although *S*. *epidermidis* is usually non-pathogenic, it is an important cause of infection in patients whose immune system is compromised, or who have indwelling catheters. Many strains produce a biofilm that allows them to adhere to the surfaces of medical prostheses. *S. epidermidis* is often resistant to a wide variety of antibiotics, including penicillin and methicillin (Mack et al, 2007, review).

Routine susceptibility testing of *Staphylococcus epidermidis* (DSMZ 20042^{T}) is performed in high throughput 96-well microtiter plate format using M218 media (BHI/3 media: 37g/L Difco BBC^{™} Brain heart infusion (BD Becton Dickinson GmbH, Heidelberg, Germany), 10 g/L NZ Amine A (Sigma-Aldrich, Taufkirchen, Germany and 1 g/L starch (Merck Biosciences GmbH, Schwalbach, Germany) in an aerobic atmosphere at 37°C. Planktonic growth of cells is determined by measuring turbidity at 580nm in a microtiter plate reader (SpectraMAX, Molecular Devices GmbH, München, Germany). Compounds dissolved in 100% DMSO are added simultaneously to an inoculum of 0.1 OD580 actively growing *S*. *epidermidis* cells to give a final concentration of 12.5µg/mL. Growth of untreated planktonic cells started to cease approximately 16 hours after inoculation resulting maximum OD580 values of approximately 0.7. Subsequently, planktonic cells are removed and the biofilm adhering at the bottom of the microtiter plate is been fixed by heat treatment (10min 80°C) and washed before staining with 0.1% crystal violet and quantified at 580nm (CV580) as described in O'Toole, G. A. and Kolter, R. (1998) in a microtiter plate reader (SpectraMAX, Molecular Devices GmbH, München, Germany). Data for planktonic proliferation and biofilm formation of *S*. *epidermidis* was collected from identical wells of a microtiter plate. Assays were done as quadruples. Biofilm development reached maximum approximately 20 hours upon inoculation. The effects of test compounds on planktonic growth of cells and formation of biofilm was determined 20-24 hours after inoculation and evaluated by comparison of turbidity (optical density, OD580) and crystal violet staining (CV580) of treated and untreated samples (control) and given as % inhibition (Figures 2 and 3).

### Example 3: Inhibitory effects on phases of population growth and biofilm formation of P. acnes.

Exponentially growing cells of *P. acnes* are in a defined active state with respect to proliferation and metabolic activity. Cell numbers are significantly higher compared to the assay described in Example 1, so that only highly active compounds will have an inhibitory effect on planktonic growth or biofilm formation of *P. acnes.* Moreover, the mode of inhibition caused by the test compound can be determined.
This assay enables to define the effectiveness of a test compound on biofilms that is not accessible with the assay set up described in Example 1. Biofilm propagation is retarded (inhibition), stopped (stagnation), or pre-formed biofilm is partially or completely dissolved (detachment). The assay can identify compounds that are active against biofilm-protected and hence persistent cells. Moreover, by combining the two read-outs of OD580 and CV580, it is possible to identify compounds selectively acting on either planktonic or biofilm cells.

For this assay *P.acnes* (DSMZ 1897^{T}) cells are grown to half-maximum planktonic cell growth and half maximum biofilm formation before test compounds are added. The relationship between optical density (OD580) and biofilm development (crystal violet staining, CV580) of *P. acnes* cells was determined previously, so that the biofilm status of the untreated control could be determined noninvasively by measuring optical density at OD580.

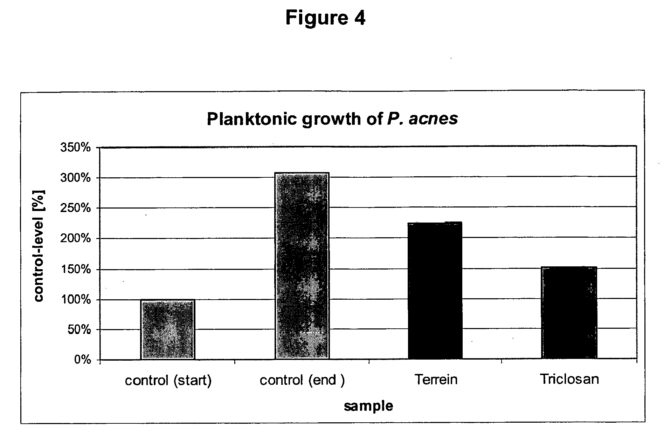

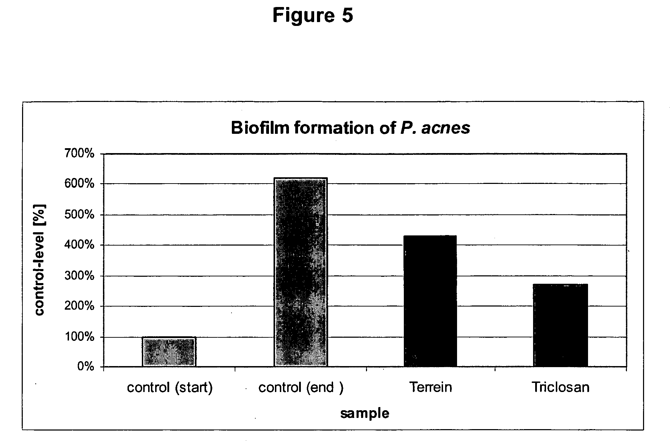

Actively growing *P. acnes* cells were seeded in Wilkins-Chalgren anaerobe broth (CM 0643, Oxoid GmbH, Wesel, Germany) in microtiter plates with an OD580 of 0.025 - 0.05 and grown at 37°C in an anaerobic atmosphere produced using the AnaeroGen^{™} system (10% CO₂ and <1% O₂, Oxoid GmbH, Wesel, Germany). After reaching approximately half maximum logarithmic growth phase (t=start), values for OD580 and CV580 were recorded and cells were incubated further until the untreated samples (control) did not show further increase in planktonic growth and/or biofilm formation (stationary phase, t=end) or test compounds, dissolved in 100% DMSO were added to a final concentration of 12.5µg/mL. Assay data points are mean value of 16 individual measurements. Numbers for OD580 and CV580 of untreated control samples and samples treated with test compounds were compared to t=start-value, which is defined as the 100% level for planktonic growth and biofilm formation, respectively. Values for OD580 and CV580 of the untreated control sample in stationary phase are defined as the "control-level" (t=end, given in %) for unrestricted growth and biofilm formation at the end of the experiment. Values for "control-levels" are solely dependent on t=start-values and therefore vary with each experiment. "Control-level" values are routinely higher than 100%, ideally show values of about 200%, but might be even higher. The effectiveness of test compounds is given as "level-%" and has to be compared to the respective "control-levels". "Level-%" values for test compounds might be close to "control-level" values (no effect), might vary between 100% and "control-level" (inhibition), might be close to 100% (stagnation), or might fall below 100 % (cell-lysis or biofilm detachment). The effects of the broad-spectrum anti-microbial agent triclosan on the planktonic growth and the biofilm formation of *P.acnes* at a final concentration of 12.5µg/mL were assayed for comparison (Figures 4 and 5).

### Further references

Burkhart, C.N. & Burkhart, C.G., Microbiology's principle of biofilms as a major factor in the pathogenesis of acne vulgaris, Int. J. Dermatol., 2003, 42, 925-927.
Bushell, D., D'Agostino, E.M., Little, J., Parry, N.J., Windust, J.H.C., Composition, patent application W02006/053613A2.
Clutterbuck, P.W., Raistrick, H.,Reuter, F. Studies in the biochemistry of micro-organisms: The molecular constitution of terrein, a metabolic product of Aspergillus terreus Thom, Biochem. J., 1937, 62, 987-1002
Del Rosso, J.Q., Combination topical therapy in the treatment of acne, Cutis, cutaneous medicine for the practitioner. 2006, 78, 5-12.
Donlan, R. M., Biofilm formation: a clinically relevant microbiological process, Clin Infect Dis. 2001, 33, 1387-1392.
Eady, E.A., Gloor, M., Leyden, J.J., Propionibacterium acnes resistance: a worldwide problem, Dermatology 2003, 206, 54-56.
He, J., Lion, U., Sattler, I., Gollmick, F.A., Grabley, S., Cai, J., Meiners, M., Schünke, H., Schaumann, K., Dechert, U., Krohn, M., Diastereomeric Quinolinone Alkaloids from the Marine-Derived Fungus Penicillium janczewskii, J. Nat. Prod., 2005, 68, 1397-1399.
Lee, T.-W., Kim, J.-C., Hwang, S.-J, Hydrogel patches containing triclosan for acne treatment, Eur. J. Pharm. Biopharm. 2003, 56, 407-412.
Lee, S., Kim, W.-G., Kim, E., Ryoo, I.-J., Lee, H.K., Kim, J.N., Jung, S.-H., Yoo, I.-D., Synthesis and melanin biosynthesis inhibitory activity of (+/-)-terrein produced by Penicillium sp. 20135, Bioorg. Med. Chem .Lett., 2005, 15, 471-473.
Malmstrøm, J., Christophersen, C., Barrero, A.F., Oltra, J.E., Justicia, J., Rosales, A., Bioactive metabolites from a marine-derived strain of the fungus Emericella variecolor, J. Nat. Prod., 2002, 65, 364-367.
Mack, D., Davies, A.P., Harris, L.G., Rohde, H., Horstkotte, M.A., Knobloch, J.K.-M., Microbial interactions in Staphylococcus epidermidis biofilms, Anal. Bioanal. Chem., 2007, 387, 399-408.
Nadell, C.D., Xavier, J.B., Levin, S.A., Foster, K.R., The evolution of quorum sensing in bacterial biofilms, PloS Biol. 2008, 6, e14.
Nenoff, P., Haustein, U.-F., Hittel, N., Activity of nadifloxacin (OPC-7251) and seven other antimicrobial agents against aerobic and anaerobic Gram-positive bacteria isolated from bacterial skin infections, Chemotherapy 2004, 50, 196-201.
O'Toole, G.A. and Kolter, R., Initiation of biofilm formation in Pseudomonas fluorescens WCS365 proceeds via multiple, convergent signalling pathways: a genetic analysis. Mol. Microbiol. 1998, 28, 449-461.
Park, S.-H., Kim, D.-S., Kim, W.-G., Ryoo, I.-J., Lee, D.-H., Huh, C.-H., Youn, S.-W., Yoo, I.-D., Park, K.-C., Terrein: a new melanogenesis inhibitor and its mechanism, Cell. Mol. Life Sci., 2004, 61, 2878-2885.
Raistrick, H. & Smith, G., Studies in the biochemistry of micro-organisms: The metabolic products of Aspergillus terreus Thom. A new mould metabolic product-terrein., Biochem. J., 1935, 29, 606-611.
Ross, J.I., Snelling, A.M., Eady, E.A., Cove, J.H., Cunliffe, W.J., Leyden, J.J., Collignon, P., Dréno, B., Reynaud, A., Fluhr, J., Oshima, S., Phenotypic and genotypic characterization of antibiotic-resistant Propionibacterium acnes isolated from acne patients attending dermatology clinics in Europe, the U.S.A., Japan and Australia, Br. J. Dermatol 2001, 144, 339-346
Yoo, I.-D., Kim, W.-G., Ryoo, I.-J., Kim, J.-P., Lee, S., Park, S.-H., Kim, D.-S., Park, K.-C., Terrein copound having melanin biosynthesis inhibitors and its preparation, 2005, patent application W02005/055995A1.

## Claims

1. A cosmetic composition for the cleaning of the skin, said composition comprising or consisting of one or more compounds of formula (I) 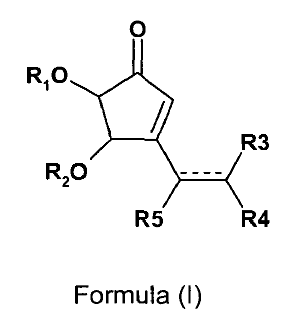 wherein
---- represents a single bond or double bond;
R₁ and R₂ are independently selected from H, linear or branched C₁ to C₄ alkyl, linear or branched C₁ to C₄ alkenyl, linear or branched C₁ to C₄ alkinyl, and linear or branched C₁ to C₄ alkanoyl;
R₄ is selected from H, linear or branched C₁ to C₄ alkyl, linear or branched C₁ to C₄ alkenyl, and linear or branched C₁ to C₄ alkinyl; and
R₃ and R₅ are independently selected from H, OH and halogen, or R₃ and R₅ together are O to form an epoxid, wherein ---- represents a single bond in case R₃ and R₅ together are O.

2. A method of cosmetic treatment comprising the administration of one or more compounds as defined in claim 1, wherein said cosmetic treatment is or comprises cleaning of the skin.

3. Use of one or more compounds as defined in claim 1 for cosmetic treatment, wherein said cosmetic treatment is or comprises cleaning of the skin.

4. The composition of claim 1, the method of claim 2 or the use of claim 3, wherein said cleaning of the skin comprises removing and/or reducing a biofilm and/or preventing the formation of said biofilm, wherein said biofilm comprises or consists of bacteria of the genus *Propionibacterium.*

5. The composition, method or use of claim 4, wherein said bacteria of the genus *Propionibacterium* are bacteria of the species *Propionibacterium acnes.*

6. A compound or a plurality of compounds, said compound(s) being selected from the compounds as defined in claim 1, for the prevention or reduction of biofilm formation on an intracorporeal device and/or for the removal of biofilm from an intracorporeal device, wherein said biofilm comprises or consists of bacteria of the genus *Propionibacterium.*

7. The compound or plurality of compounds of claim 6, wherein said intracorporeal device is selected from catheters, implants, endoscopes, drainages, contact lenses and hearing aids.

8. The compound or plurality of compounds of claims 6 or 7, wherein said bacteria of the genus *Propionibacterium* are bacteria of the species *Propionibacterium acnes.*

9. A method of preventing or reducing formation of a biofilm on a device for intracorporeal use and/or of removing of a biofilm from a device for intracorporeal use, wherein said device is not present in a human or animal body, and wherein said biofilm comprises or consists of bacteria of the genus *Propionibacterium,* said method comprising bringing said device into contact with a compound or a plurality of compounds, said compound(s) being as defined in claim 1.

10. Use of a compound or a plurality of compounds, said compound(s) being as defined in claim 1 for preventing or reducing formation of a biofilm on a device for intracorporeal use and/or for removing of a biofilm from a device for intracorporeal use, wherein optionally said device is not present in a human or animal body, and wherein said biofilm comprises or consists of bacteria of the genus *Propionibacterium.*

11. The method of claim 9 or the use of claim 10, wherein said device for intracorporeal use is selected from catheters, implants, endoscopes, drainages, contact lenses and hearing aids.

12. The method of claim 9 or 11 or the use of claim 10 or 11, wherein said bacteria of the genus *Propionibacterium* are bacteria of the species *Propionibacterium acnes.*

13. An intracorporeal device which is coated and/or loaded with one or more compounds, said compound(s) being as defined in claim 1.
